# EUROPEAN PATENT APPLICATION

(11) **EP 1 669 086 A1**
(43) Date of publication of application: **14.06.2006**
(21) Application number: 05002383.7
(22) Date of filing: 04.02.2005
(51) Int. Cl.: A61K 38/18, A61K 35/20, A61P 17/06

(54) **Composition comprising TGF-beta and proteins for treating psoriasis**

(30) Priority: 09.12.2004 US 7912; 09.12.2004 CA 2489851
(71) Applicant: Advitech Solutions Inc., Québec Quebec, G2K 2C9 (CA)
(72) Inventor: Pouliot, Yves, Charny Quybec, G6X 3P3 (CA); Gauthier, Sylvie, Charny Quybec, G6X 3P3 (CA); Lamiot, Eric, Quybec Quybec, G1J 1T4 (CA); Aattouri, Najat, Quybec, G6K 1S7 (CA); Juneau, Christina, Pont-Rouge Quybec, G3H 2A2 (CA)
(74) Representative: Modiano, Micaela Nadia

(57) **Abstract**

The present invention relates to a composition and methods or uses thereof in the prophylaxis and treatment of psoriasis and related disorders. The composition may be comprised of TGF-β1, TGF-β2, and a large proportion of dairy derived proteins.

## Description

### BACKGROUND OF THE INVENTION

### a) Field of the invention

The present invention relates in general to the field of the treatment of psoriasis and related skin disorders, and more particularly to a non-toxic oral and topical formulation that includes a pharmaceutically effective amount of dairy proteins for the treatment of psoriasis and related immune system disorders.

### b) Description of the prior art

It is not known what causes psoriasis, although there is evidence of a genetic predisposition and an autoimmune etiology. Onset may be triggered by systemic infections such as streptococcus throat, skin injury, vaccinations, and certain oral medications such as steroids. Subsequently, the immune system is thought to induce inflammation and excessive skin cell reproduction, which can be exacerbated by additional factors such as stress and diet.

In normal skin, the time for a cell to move from the basal layer through the granular layer is 4-5 weeks. In psoriatic lesions, the time is decreased 7-10 fold because of a shortened cell cycle time, an increase in the absolute number of cells capable of proliferating, and an increased rate of division. T cell mediated immune responses appear to be responsible for the inflammation and hyperproliferation of keratinocytes. Neutrophils are found in psoriatic lesions, associated with increased levels of plasminogen activator. Psoriatic fibroblasts have increased levels of enzymes involved in collagen synthesis, secondary to expansion of the papillary dermis. Psoriatic plaques comprise HLA-DR positive keratinocytes and Langerhans cells, and activated T cells expressing elevated levels of IL-2 receptors.

The typical lesion of psoriasis is a well-demarcated erythematous plaque, covered by thick, silvery scales. Psoriasis can become so extensive as to cause exfoliative erythroderma, in which the entire epidermal surface is in a state of hyperproliferation. Gluttate psoriasis is a form of the disease following streptococcal pharyngitis, with widely distributed characteristic 1-3 cm lesions. Pustular psoriasis is characterized by numerous sterile pustules of 2-5 mm in diameter, and may lead to an acute, explosive, life-threatening episode of fever, chills, leukocytosis, hypoalbuminemia, and hypocalcemia, demanding immediate, vigorous therapy. Previously stable plaque-type psoriasis can be acutely exacerbated by viral infections, particularly HIV. Psoriasis is also associated with five different forms of psoriatic arthritis, including distal interpharangeal involvement; an asymmetric, oligoarticular pattern; a symmetric polyarthritis; arthritis mutilans; and sacroiliitis and spondylitis.

The inflammation and hyperproliferation of psoriatic tissue is associated with a different histological and antigenic profile than normal skin. A panel of anti-carbohydrate monoclonal antibodies as described in the art, can be used to compare psoriatic tissue with the surrounding dermis. The glycosylation pattern in psoriatic epithelium is changed in two ways: some carbohydrates are expressed at an earlier stage of cell maturation. In addition, certain biosynthetic precursor antigens not expressed in normal skin were found in psoriatic skin.

Classical treatments of psoriasis include calcipotriene (a vitamin D₃ derivative), topical coal tar preparations, systemic antimitotic agents such as methotrexate, and retinoids, particularly etretinate.

Extensive psoriasis can be treated by photosensitization with oral 8-methoxypsoralen, followed by ultraviolet A. Corticosteroids are given for psoriatic arthritis and acute attacks of pustular psoriasis. More recently, cyclosporin A has been tested in clinical trials at doses of 3-7 mg/kg with promising results, but associated with the risk of renal toxicity.

Current biotechnology approaches to psoriasis treatment relate to a direct pharmaceutical-mediated attack, either on cell proliferation or on the immune component of the disease. Japanese patent application JP 6145069 describes angiogenesis inhibitors comprising ganglioside GM3 or a GM3 analog as an active agent. At 100 µg/mL, GM3 showed growth of normal human anti-endothelial cells of 4.5.times. 10⁴ on day 5, compared with 76.times.10⁴ in controls. U.S. Pat. No. 5,339,977 describes n-deacetyl-lysoganglioside derivatives for use as phospholipase A2 inhibitors for the treatment of proliferative and autoimmune diseases, including various forms of cancer, psoriasis, and rheumatoid arthritis.

An IL-2 fusion toxin has been developed (Seragen, Inc.) that is designed to selectively destroy activated T cells in psoriatic plaques, leaving normal cells alone. The objective is to destroy activated T cells, and thereby clear the psoriasis. A Phase II study has been performed in which test doses of 5, 10, and 15 µg/kg were administered per day. Comparable improvement was observed in patients with moderate to severe psoriasis. However, in order to obtain this response, the compound was administered three days per week for four weeks.

Various formulations containing the compound BCX-34 for psoriasis, cutaneous T cell lymphoma, and HIV infection have been tested (Bioworld Today, Sep. 29, 1997; see also WO 95/01355; WO 93/21187; WO 90/10631; U.S. Pat. Nos. 5,008,270, 5,008,265, and 4,985,434). BCX-34 is a small molecule drug that inhibits purine nucleoside phosphorylase, a human enzyme believed to be involved in the proliferation of T cells. An oral formulation is being tested in an ongoing Phase I/II trial. A topical formulation advanced to the Phase III stage for both lymphoma and psoriasis. The Phase III psoriasis study showed only a 14% greater improvement in mean lesion scores in the treated group compared to placebos, which for these studies was not statistically significant.

Several drugs have been designed to treat psoriasis by targeting specific cells, specific cytokines, or specific interactions between ligands and receptors. The main advantage of these biological agents over cyclosporin and methotrexate is the absence of nephrotoxicity and hepatotoxicity, but toxic effects can take years to develop. Candidates for biological therapy will be those who cannot comply with the rigours of a phototherapy regimen or have received too much PUVA and are at risk of hepatotoxicity and nephrotoxicity.

Another product available in the art consist in a chimeric human tumor necrosis factor α monoclonal antibody derived from mouse. It is made of the human constant and mouse variable regions of the IgG antibody. This agent is administered by intravenous infusion over at least 2 h and neutralizes soluble tumour necrosis factor α bound to cell membranes. Results of several case reports attest to the efficacy of this agent in treatment of psoriasis. Drawbacks of the drug include the need for slow intravenous infusion. A small but real proportion of patients have infusion reactions, including potentially serious reactions such as hypotension, rigors, and allergic reactions. These reactions can often be prevented by slowing down the infusion or pre-treating with antihistamines or, for some conditions, systemic corticosteroids. Neutralizing antibodies can develop, making the treatment less effective. Worse, patient who develop neutralizing antibodies are more likely to develop infusion reactions. One of the greatest concerns is the potential for infection with this tumor necrosis factor α inhibitor.

Alternatively, recombinant tumour necrosis factor α receptor fusion proteins were developed, which consist of two extracellular ligand-binding domains of the human p75 tumour necrosis factor α receptor fused to the Fc portion of human IgGl. These fusion proteins are likely effective for psoriatic arthritis and seems to be safer than ciclosporin or methotrexate with no nephrotoxicity or hepatotoxicity. Non-neutralising antibodies occur in less than 5% of patients. Antinuclear antibodies and antibodies to double-stranded DNA have been reported, but full cases of systemic lupus erythematosus are rare. Anticardiolipin antibodies also develop but have been attributed to minor concomitant infections. Because this agent is a tumour necrosis factor α inhibitor, concern has been raised about the potential for immunosuppression leading to infection.

Humanised anti-CD11 a monoclonal antibody was also developed for the treatment for moderate-to-severe plaque psoriasis and moderate-to-severe rheumatoid arthritis. It is normally administered as a subcutaneous (under the skin) injection, and is designed to inhibit the binding of immune system T-cells to other cell types and targets three key processes in the cascade of events that lead to autoimmune symptoms.

Another family of products is known in the art to prevent T cell activation by blocking the LFA-3/CD-2 pathway through binding to the CD2 receptor. It is an IV/IM administered product. Since this family of products may cause reduction in CD4+ and CD8+ T lymphocyte counts, it may not be appropriate for all individuals.

A fully human fusion protein consisting of a binding site of LFA-3 fused to the Fc portion of IgG1 CD45Ro+ memory T cells, which have a major role in development of psoriasis, maximally express CD2, a natural ligand of LFA-3 has been developed. By binding CD2, it prevents T-cell activation. Moreover, the Fc portion of the molecule engages Fc receptors on macrophages and NK cells, which results in apoptosis of the CD45RO+ T cells.

Another humanised monoclonal antibody to CD11A was developed, which is a component of LFA-1 on T cells and ICAM-1 on antigen-presenting cells is an important co-stimulatory signal resulting in T-cell activation. ICAM-1 on endothelial cells also interacts with LFA-1 on circulating T cells, a necessary step for migration of T cells into inflamed skin. It can thus interfere with development of psoriasis by blocking T-cell migration into the skin and by preventing T-cell activation. In clinical trials, some patients developed flu-like symptoms including headache, chills, fever, nausea, vomiting, or myalgias. Symptoms arose on the day of injection or the following 2 days. These acute adverse events subsided by the third dose.

Systemic treatment has been used in patients with physically, socially, or economically disabling psoriasis that has not responded to topical treatment. The choices to date have been phototherapy or systemic drug therapy. Generally, systemic treatment has employed phototherapy with Ultraviolet B irradiation, photo chemotherapy which combines the photosensitizing drug methoxsalen with Ultraviolet A phototherapy (PUVA), methotrexate, etretinate, systemic corticosteroids, and cyclosporine. Each of these systemic treatments has variable efficacy and undesired side effects, and some of them are very toxic and present frequent relapses of the disease.

The number of different and sometimes toxic treatments employed for amelioration of psoriasis is testimony to the resistant nature of this disease. Not only is moderate to severe psoriasis resistant to topical treatments, but because of its chronic and recurrent nature, systemic therapy or radiation is often required. The devastating nature of this disease is emphasized by the extent of the side effects that psoriasis sufferers are willing to endure to attain a remission to a disease that they know will recur sooner or later.

Keratinocyte function is regulated via intracellular signalling pathways triggered by growth factors and adhesion molecules. Among them, the EGF family and the TGF-β family are thought to play central roles; they provide dual mode regulation of keratinocytes growth via the proliferation stimulating effect of EGF and the proliferation-inhibiting effect of TGF-β. TGF-βs exert a wide range of biological effects on keratinocytes, such as growth inhibition, production of extracellular matrix, and synthesis of plasminogen activator and its inhibitor (PAI 1). Among them, growth inhibition is the most prominent.

TGF-β1 and TGF-β2 are synthesized and secreted in human keratinocytes. It seems that TGF-β3 is not a major TGF-β in human keratinocytes growth. Vitamin D3 is a strong inhibitor keratinocyte growth. The involvement of TGF-β production induced by vitamine D3 increased the expression of TGF-β2 mRNA 4 to 5 fold. In contrast, TGF-β1 mRNA and TGF-β3 mRNA were not increased. Taken together, these data suggest that the intrinsic TGF-βs regulate autonomous growth of human keratinocytes, and have demonstrate that TGF-β antagonized the acanthotic and degenerative effect of TGF-α in involved psoriatic skin, but TGF-β alone did not cause granular layers to appear in the involved psoriatic epidermis, indicating that TGF-β alone cannot normalize the psoriatic condition of the epidermis.

TGF-β is now recognized as a potent growth inhibitor for human keratinocytes. TGF-β2, which was found through the normal human epidermis, was decrease in the proriatic epidermis. Since TGF-β is a strong growth inhibitor for human keratinocytes this result indicates the possibility that the decrease of TGF-β2 is involved in the pathogenesis of psoriasis. Therefore, the decrease in TGF-β2 in psoriasis epidermis may induce the accelerated proliferation of keratinocytes and result in epidermal hyperplasia. TGF-β is also a potent immunosuppressive agent. So the decrease of TGF-β2 may permit propagation of an immune/inflammatory reaction in the dermis and epidermis of involved psoriasis. It has been supposed that an immune-activation triggers the growth activation of epidermal keratinocytes in psoriasis. In terms of growth regulation of keratinocytes, an involvement of two groups of growth factors and cytokines, proliferation-stimulating growth factors and proliferation-inhibitory growth factors, has been reported. EGF family growth factors (TGF-α, amphiregulin and HB-EGF), KGF and IL-6 are well known as proliferation-stimulating growth factors. TGF-β family growth factors are the major proliferation-inhibitory growth factors. The increase of TGF-α, amphiregulin and IL-6 was found in psoriasis. Autocrine- or cross-induction of EGF family growth factors may play an important role in epidermal hyperplasia.

One important group of growth factors involved in psoriasis mechanisms are the dermally-derived insulin-like growth factors (IGFs), which support keratinocyte proliferation. In particular, IGF-I and IGF-II are ubiquitous peptides each with potent mitogenic effects on a broad range of cells. Molecules of the IGF type are also known as "progression factors" promoting "competent" cells through DNA synthesis. The IGFs act through a common receptor known as the Type I or IGF-I receptor, which is tyrosine kinase linked. They are synthesized in mesenchymal tissues, including the dermis, and act on adjacent cells of mesodermal, endodermal or ectodermal origin. The regulation of their synthesis involves growth hormone (GH) in the liver, but is poorly defined in most tissues.

Particular proteins, referred to as IGF binding proteins (IGFBPs), appear to be involved in autocrine/paracrine regulation of tissue IGF availability. Six IGFBPs have so far been identified. The exact effects of the IGFBPs is not clear and observed effects *in vitro* have been inhibitory or stimulatory depending on the experimental method employed. There is some evidence, however, that certain IGFBPs are involved in targeting IGF-I to its cell surface receptor.

Skin, comprising epidermis and underlying dermis, has GH receptors on dermal fibroblasts. Fibroblasts synthesize IGF-I as well as IGFBPs-3, -4, -5 and -6, which may be involved in targeting IGF-I to adjacent cells as well as to the overlaying epidermis. The major epidermal cell type, the keratinocyte, does not synthesize IGF-I, but possesses IGF-I receptors and is responsive to IGF-I.

Considering the state of the art described above, there is still a significant need for an effective psoriasis treatment that avoids the disadvantages associated with the currently available topical or systemic treatments.

### SUMMARY OF THE INVENTION

One aim of the present invention is to provide a composition and uses thereof for the prophylaxis or treatment of psoriasis or related disorders, said composition comprising between 0.1 to 5 µg of TGF-β1 and between 5 to 50 µg of TGF-β2 per gram of composition, and a total protein concentration completed to at least 15% (w/w), but preferably to at least 50% , and most preferably 80%, with dairy derived proteins. TGF-β1 and TGF-β2 can also be dairy derived, and can be found at concentration between 0.2 to 1.2 µg/g and between 10 to 18 µg respectively per gram of composition.

The composition may comprises additionally between about 0.1 to 15% of minerals as defined herein after.

At least 50% of the proteins are hydrosoluble. Preferably, 70%, and most preferably 80% of the proteins found in the composition are hydrosoluble. The ratio of TGF-β1+TGF-β2/IGF-1 is preferably of between about 0.1 to 3.

Someone skilled in the art will recognize that types of psoriasis that can be prevented or treated with the method or composition of the present invention may include one of nail psoriasis, plaque psoriasis, guttate psoriasis, erythrodermic psoriasis, pustular psoriasis, hyperkeratosis, oncholysis, or psoriatic arthritis.

Another aim of the present invention is to provide a method for preventing or treating psoriasis or related disorders comprising administering to a patient between 0.1 to 5 µg of TGF-β1 and between 5 to 50 µg of TGF-β2 per gram of composition, and a total protein concentration completed to at least 80% (w/w) with dairy derived proteins.

Accordingly, one aspect of the present invention contemplates a method for ameliorating the effects of a proliferative and/or inflammatory skin disorder associated with psoriasis in a mammal, the method comprising treatment of the psoriasis and related diseases with an effective amount of a composition or chemical analogues thereof capable of having properties in preventing or treating psoriasis and related diseases.

For the purpose of the present invention the following terms are defined below.

The term "psoriatic tissue" refers to tissue affected by psoriasis and affected cells contained within the tissue, but not to cells that have migrated to the site such as leukocytes. Preferably, the psoriatic tissue is from a human.

The expression "effective amount" as used herein is intended to mean an amount sufficient to effect a beneficial or desired clinical result. An effective amount can be administered in one or more doses. For purposes of this invention, an effective amount of growth factors and/or dairy derived proteins, or other composition is an amount that induces a treatment or prophylactic response against at least one psoriasis responsible factor.

The terms "polypeptide", "peptide" and "protein" are used interchangeably to refer to polymers of amino acids of any length, and may be interrupted by non-amino acids.

The terms "individual" or "subject" treated according to this invention is a vertebrate, preferably a mammal, more preferably a human. Mammals include, but are not limited to, farm animals, sport animals, rodents, primates, and pets.

The term "minerals" as used herein is intended to mean salt constituents and minerals at concentrations normally found in dairy products. For example, there can be different concentration of calcium, phosphorus, magnesium, potassium, sodium, chloride, sulfur, and citric acid. This also means that trace elements known in the art as found in dairy products can be assimilated or inferred therein.

Other terms used in this disclosure are explained where they arise.

### BRIEF DESCRIPTION OF THE DRAWINGS

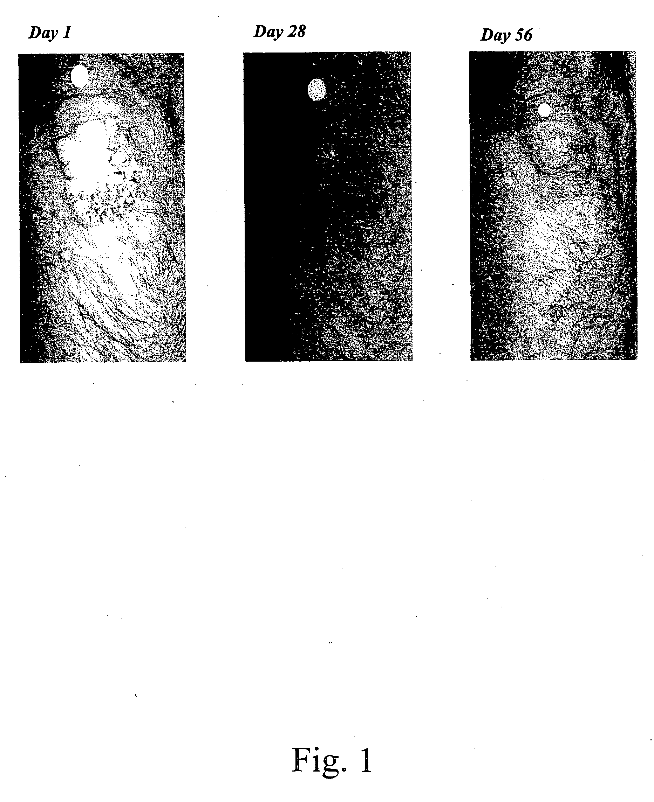

Fig. 1 illustrates knee skin of patient no. 4 after 1, 28 and 56 days of treatment with the composition XL-828L according to one embodiment of the present invention;

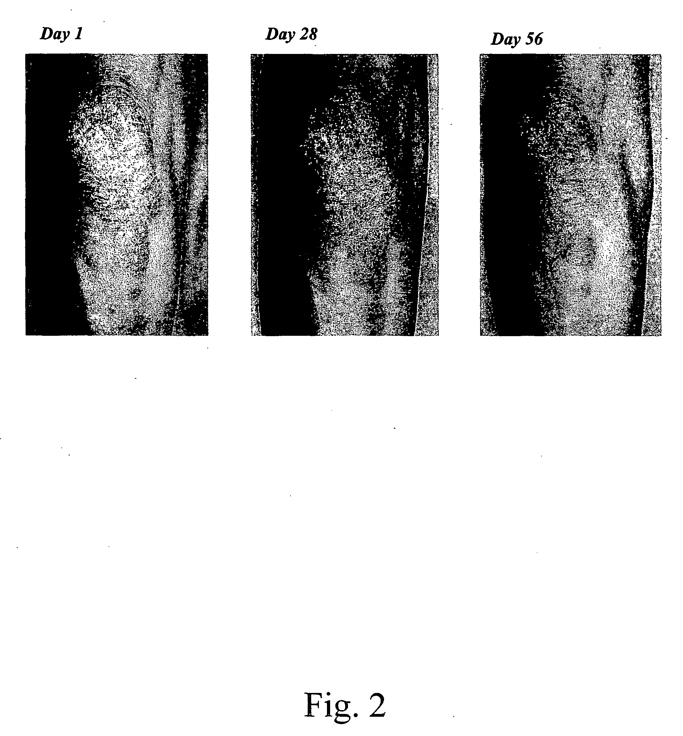

Fig. 2 illustrates knee skin of patient no. 9 after 1, 28 and 56 days of treatment with the composition XL-828L according to one embodiment of the present invention; and

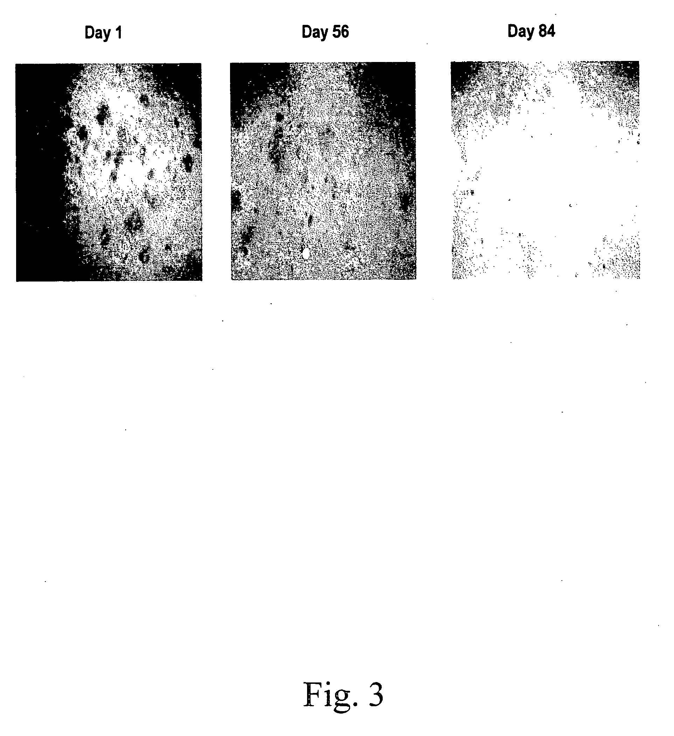

Fig. 3 illustrates knee skin of patient no. 4 after 1, 56 and 84 days of treatment with the composition XL-828L according to one embodiment of the present invention;

### DESCRIPTION OF THE PREFERRED EMBODIMENT

The present invention now will be described more fully hereinafter with reference to the accompanying drawings, in which preferred embodiments of the invention are shown. This invention, may, however, be embodied in many different forms and should not be construed as limited to the embodiments set forth herein; rather, these embodiments are provided so that this disclosure will be thorough and complete, and will fully convey the scope of the invention to those skilled in the art.

All patents, patent applications, articles and publications mentioned herein, both supra and infra, are hereby incorporated herein by reference.

In accordance with the present invention, there is provided composition and methods for treating psoriasis in an individual, comprising administering a composition effective in stimulating a specific immunological response against a physiological imbalance aberrantly expressed in psoriatic tissue. These composition(s) comprise a cocktail of products that shares growth and regeneration characteristics of a growth factor that is aberrantly expressed in psoriatic tissue (such as human psoriatic tissue). Particular growth factors included in the composition of the present invention, but are not limited to transforming growth factors, such as TGF-β1 and TGF-β2, and at least 15% of dairy derived proteins. Preferably, the composition of the present invention will comprised of at least 40% or 50% of dairy derived proteins, and most preferably more than 80% of dairy derived proteins. In one particular embodiment of the present invention, at least 70%, and preferably 80%, of the proteins are hydrosoluble. The proteins may be comprised, for example but not limited to, of at least 60% (w/w) of β-lactoglobulin, and/or between 0.1 to 30% (w/w) of lactoferrin. Milk proteins are naturally presents as follows:

| | | grams/ litre | % of total protein |
|---|---|---|---|
| Total Protein | | 33 | 100 |
| Total Caseins | | 26 | 79.5 |
| | alpha s1 | 10 | 30.6 |
| | alpha s2 | 2.6 | 8.0 |
| | beta | 9.3 | 28.4 |
| | kappa | 3.3 | 10.1 |
| Total Whey Proteins | | 6.3 | 19.3 |
| | alpha lactalbumin | 1.2 | 3.7 |
| | beta lactoglobulin | 3.2 | 9.8 |
| | BSA | 0.4 | 1.2 |
| | Immunoglobulins | 0.7 | 2.1 |
| | Proteose peptone | 0.8 | 2.4 |

### Whey Proteins

The proteins appearing in the supernatant of milk after precipitation at pH 4.6 are collectively called whey proteins. These globular proteins are more water soluble than caseins and are subject to heat denaturation. Native whey proteins have good gelling and whipping properties. Denaturation increases their water holding capacity. The principle fractions are β -lactoglobulin, alpha-lactalbumin, bovine serum albumin (BSA), and immunoglobulins (Ig).

β -Lactoglobulins: (MW - 18,000; 162 residues) This group, including eight genetic variants, comprises approximately half the total whey proteins. β -Lactoglobulin has two internal disulfide bonds and one free thiol group. The conformation includes considerable secondary structure and exists naturally as a noncovalent linked dimer. At the isoelectric point (pH 3.5 to 5.2), the dimers are further associated to octamers but at pH below 3.4, they are dissociated to monomers.

alpha-Lactalbumins: (MW - 14,000; 123 residues) These proteins contain eight cysteine groups, all involved in internal disulfide bonds, and four tryptophan residues. alpha-Lactalbumin has a highly ordered secondary structure, and a compact, spherical tertiary structure. Thermal denaturation and pH <4.0 results in the release of bound calcium.

### Enzymes

Enzymes are a group of proteins that have the ability to catalyze chemical reactions and the speed of such reactions. The action of enzymes is very specific. Milk contains both endogenous and exogenous enzymes. Exogenous enzymes mainly consist of heat-stable enzymes produced by psychrotrophic bacteria: lipases, and proteinases. There are many endogenous enzymes that have been isolated from milk. The most significant group are the hydrolases: lipoprotein lipase; plasmin; and alkaline phosphatase.

Lipoprotein lipase (LPL): A lipase enzyme splits fats into glycerol and free fatty acids. This enzyme is found mainly in the plasma in association with casein micelles. The milk fat is protected from its action by the fat globular matrix (FGM). If the FGM has been damaged, or if certain cofactors (blood serum lipoproteins) are present, the LPL is able to attack the lipoproteins of the FGM. Lipolysis may be caused in this way.

Plasmin: Plasmin is a proteolytic enzyme; it splits proteins. Plasmin attacks both β-casein and alpha(s2)-casein. It is very heat stable and responsible for the development of bitterness in pasteurized milk and UHT processed milk. It may also play a role in the ripening and flavour development of certain cheeses, such as Swiss cheese.

Alkaline phosphatase: Phosphatase enzymes are able to split specific phosporic acid esters into phosphoric acid and the related alcohols. Unlike most milk enzymes, it has a pH and temperature optima differing from physiological values; pH of 9.8. The enzyme is destroyed by minimum pasteurization temperatures, therefore, a phosphatase test can be done to ensure proper pasteurization.

While a detectable immunological or tissue response is likely to be beneficial, efficacy can also be deduced by an improvement in symptoms or control of the psoriatic condition beyond what would be expected without treatment.

The active compounds according to the invention are distinguished by strong antiinflammatory actions.

For prophylaxis, the active compounds are administered in order to decrease manifestations of the disease in frequency and strength. Treatment in the manifest stage leads to its curtailment and to the alleviation of the symptoms.

In all types of psoriasis, the active compounds of the present composition can be used prophylactically and for the treatment of the disorders.

Estimates of the prevalence of psoriasis vary from 0,5 to 6%, with rates varying between countries and races. Different types of psoriasis can be prevented or treated with the composition of the present invention. Plaque-type psoriasis is the most common form of the disease, occurring in more than 80% of cases. Guttatte psoriasis occurs in about 10% of patients with psoriasis, and erythrodermic and pustular psoriasis each occur in fewer than 3% of patients. Nail psoriasis is generally the first sign of disease in 4% of patients. Between 5 to 10% of patients with psoriasis have psoriatic arthritis, a destructive and occasionally disabling joint disease. The course of psoriatic arthritis varies, with some having mild changes and others severe, rapid destruction of joints. Skin problems such as dryness or lesions are symptoms associated with psoriasis.

Erythrodermic psoriasis is characterized by generalized inflamed erythema and widespread scaling, affecting up to 100% of the body surface area. Patients lose many of the protective functions of the skin, including the skin's ability to protect against infection, control body temperature, and prevent loss of fluids and nutrients through the cutaneous surface.

Generalized pustules psoriasis is characterized by development of sterile pustules covering large portions of the trunk and extremities. In severe cases, pustules become confluent forming large areas of pus. In this case also, many of the skin's protective functions are lost, making patients susceptible to infection and loss of fluids and nutrients.

A further neurosensory phenomenon is to be regarded as itching in the case of atopic skin, and also itching in the case of psoriasis related disorders.

According to the invention, it is therefore possible to make available active compounds and preparations containing those active compounds which, in particular, prevent neurosensory phenomena or alleviate them or rapidly make them fade, i.e. are suitable for prophylaxis and/or treatment.

"Stinging" phenomena can be regarded as disorders to be treated cosmetically or orally, or in other cases by other ways of administration. Severe itching, however, in particular in the case of atopy, in particular neurodermatitis and severe itching of the skin occurring during psoriasis, can also be regarded as a relatively serious dermatological disorder.

The active compounds according to the invention can in particular also be used on skin superficially appearing to be healthy, e.g. in the case of psoriasis and atopy, i.e. also in addition to the diseased skin areas and, in particular, here too in the case of related psoriasis disorders.

The active compounds according to the invention can also be incorporated without problems into customary oral, pharmaceutical, dermatological, and cosmetic bases for preferred oral administrations and the corresponding pharmaceutical, in particular dermatological, and cosmetic topical preparations or compositions can thus be obtained. The concentration of compounds in the composition of the present invention are adjusted depending of the needs. TGF-β1 can be found at concentration of between 0.1 to 5 µg per gram of composition and TGF-β2 can be found at concentration of between 5 to 50 µg per gram of composition. Preferably, TGF-β1 is found at concentration of 0.2 to 1.2 µg/g, and TGF-□2 at between 10 to 18 µg/g of composition. The preparations can be used daily in a customary manner.

In another embodiment of the present invention, the composition comprises at least 70% (w/w) of dairy derived proteins. The total concentration of proteins in the composition would normally of at least 80%. Dairy proteins or dairy products as used herein may include milk, colostrums, or whey proteins or derived products or fractions thereof. The fat fraction of the composition can be generally found at concentration between about 0.5 to 10 % (w/w) in the composition. Though not always the case, the fat or compounds of this family of products, can be derived from dairy products, or from any other source, such as for example, but not limited to, vegetable or animal sources, synthetic or natural sources, allowing the composition of the present invention to have its properties as defined herein. For example, the physical properties of milk fat can be summarized as follows: density at 20°C is 915 kg/m³; refractive index (589 nm) is 1.462 which decreases with increasing temperature; solubility of water in fat is 0.14% (w/w) at 20°C and increases with increasing temperature; thermal conductivity is about 0.17 J/m/s/K at 20°C; specific heat at 40°C is about 2.1kJ/kg/K; electrical conductivity is <10⁻¹²/ ohm/cm; and dielectric constant is about 3.1.

At room temperature, the lipids are solid, therefore, are correctly referred to as "fat" as opposed to "oil" which is liquid at room temperature. The melting points of individual triglycerides ranges from -75°C for tributyric glycerol to 72°C for tristearin. However, the final melting point of milk fat is at 37°C because higher melting triglycerides dissolve in the liquid fat. This temperature is significant because 37°C is the body temperature of the cow and the milk would need to be liquid at this temperature. The melting curves of milk fat are complicated by the diverse lipid composition: trans unsaturation increases melting points; and odd-numbered and branched chains decrease melting points.

The invention also relates to the use of the active compounds according to the invention for the production of pharmaceutical compositions, in particular oral, pharmaceutical and cosmetic compositions for the prophylaxis and treatment of psoriasis, allergies and auto-immune disorders and on dry skin and on sensitive skin.

Likewise, the invention also relates to the use of the compositions as oral, or topical preparations.

The present invention also addresses the underlying T-cell disorder that results in an inflammatory condition due to psoriasis. The present inventors have recognized that most, if not all, of the current therapies for psoriasis or similar T-cell mediated inflammatory skin conditions are designed to eliminate T-cells and to thereby ameliorate inflammation. It is possible that a major problem with the current treatments is that the therapy itself is so toxic that it may promote recurrence during healing. The toxicity of current treatments unleashes some or all of the cytokines that are associated with the promulgation of these chronic and often rebounding skin diseases.

The synergistic effects of the two TGF-β factors and dairy derived proteins results in a non-toxic, highly effective treatment for psoriasis that is without the side effects observed with virtually all other therapies for moderate to severe psoriasis (mild psoriasis may be successfully treated with proper moisturizing). As illustrated by the following studies, the most common over-the-counter treatment for psoriasis, namely coal tar, may be made more effective by the use of the present composition.

The active compounds according to the invention can be mixed with customary pharmaceutically tolerable diluents or vehicles and, if appropriate, with other auxiliaries and administered, for example, orally or parenterally. They can preferably be administered orally in the form of granules, capsules, pills, tablets, film-coated tablets, sugar-coated tablets, syrups, emulsions, suspensions, dispersions, aerosols and solutions and also liquids, or else also as suppositories, vaginal suppositories or parenterally, e.g. in the form of solutions, emulsions or suspensions. Preparations to be administered orally can contain one or more additives such as sweeteners, aromatizing agents, colorants and preservatives. Tablets can contain the active compound mixed with customary pharmaceutically tolerable auxiliaries, for example inert diluents such as calcium carbonate, sodium carbonate, lactose and talc, granulating agents and agents which promote the disintegration of the tablets on oral administration, such as starch or alginic acid, binding agents such as starch or gelatin, lubricants such as magnesium stearate, stearic acid and talc.

Suitable excipients are, for example, lactose, gelatin, maize starch, stearic acid, ethanol, propylene glycol, ethers of tetrahydrofurfuryl alcohol and water.

The formulations are prepared, for example, by extending the active compounds with solvents and/or excipients, if appropriate using emulsifiers and/or dispersants, it being possible, for example, in the case of the use of water as a diluent optionally to use organic solvents as auxiliary solvents.

Administration is carried out in a customary manner, preferably orally or parenterally, in particular perlingually, sublingually, or intravenously. In the case of oral administration, apart from the excipients mentioned, tablets, of course, can also contain additives, such as sodium citrate, calcium carbonate and dicalcium phosphate together with various additives, such as starch, preferably potato starch, gelatin and the like. Furthermore, lubricants such as magnesium stearate, sodium lauryl sulphate and talc can additionally be used for tableting. In the case of aqueous suspensions and/or elixirs, which are intended for oral administration, the active compounds can be mixed, apart from with the above-mentioned auxiliaries, with various flavors enhancers or colorants.

In the case of parenteral administration, solutions of the active compounds using suitable liquid excipients can be employed.

Capsules can contain the active compound as a single constituent or mixed with a solid diluent such as calcium carbonate, calcium phosphate or kaolin. The injectable preparations are also formulated in a manner known *per se*.

Ointments and topical formulations are particularly of interest also when considering the use of the composition of the present invention for treating psoriasis.

The preferred compositions according to the invention can be formulated as liquid, pasty or solid preparations, for example as aqueous or alcoholic solutions, aqueous suspensions, emulsions, for example W/O or O/W emulsions, ointments, gels, lotions, creams, oils, powders or sticks. Depending on the desired formulation, the active compounds can be incorporated into pharmaceutical and cosmetic bases for topical application, which as further components contain, for example, oil components, fat and waxes, emulsifiers, anionic, cationic, ampholytic, zwitterionic and/or non-ionic surfactants, lower mono- and polyhydric alcohols, water, preservatives, buffer substances, thickeners, fragrances, colorants and opacifiers. Preferably, the emulsions, e.g. W/O emulsions, or ointments are used.

Furthermore, it is preferred according to the invention to add antioxidants to the active compounds and to the pharmaceutical and topical preparations. The use of natural or naturally identical compounds such as, for example, tocopherols is particularly preferred here. The antioxidants mentioned are contained in the compositions according to the invention, for example, in amounts from 0.01-5% by weight, in particular from 0.5-2% by weight, based on the total composition.

The present invention will be more readily understood by referring to the following examples which are given to illustrate the invention rather than to limit its scope.

### EXAMPLE I

### Clinical efficiency of oral treatment.

### Materials and Methods

### Protocol design

The study was carried out in de "Centre de Recherche Dermatologique du Québec Métropolitain". The study protocol was approved by an external ethic committee (Ethica Clinical Research inc., Montreal). All patients were submitted to a washout period of 28 days for the systemic therapies and 14 days for the topic therapies (including phototherapy) before the beginning of the study. Only tar or salicylic acid based shampoos for the scalp, mild topic corticosteroids for genital and facial areas, and an hydrating cream provided by the clinic were permitted during the washout and the treatment periods.

During the initial 56-day treatment period, patients received one pouch (5g) of XP-828L twice a day. For the remaining of the study, the product provided by the sponsor was concentrated by a factor 2X in terms of growth factors. Therefore only 2.5g of XP-828L were put in the pouches in order to administer the equivalent amount of growth factors on a daily basis, i.e. 60 µg of active ingredient. All patients participating at the extension study received the new lot and were informed of the introduction of the new product in the protocol.

All patients provided written informed consent. Eligible patients were at least 18 years of age and diagnosed as having active plaque psoriasis with at least 2 % of total body surface area affected.

### Assessments

Patients were evaluated at screening, baseline and at days 28, 56, 84 and 112 of treatment. The assessments performed at each visit are summarized on Table 1.

### Psoriasis

The score for the psoriasis area-and-severity index (PASI) was the primary outcome measured by the physician. The PASI combines assessments of psoriasis induced erythema, scaling and skin thickness, each weighted according to the size of the affected area. The composite score ranges from 0 to 72.

At each visit, the physician performed the Physician's Global Assessment (PGA), which is a general evaluation of the patient's psoriasis. The PGA uses a 5-point scale: very severe (5); severe (4); moderate (3); mild (2); almost clear (1) and absence of disease (0) (Table 1).

Patients also evaluated their psoriasis using the Patient's Global Assessment (Patient GA, 10-point scale) and the Pruritus Assessment (4-point scale).

The percentage of Body Surface Area (BSA) affected by psoriasis was also evaluated.

Pictures of a specific area were taken at each visit.

### Evaluation of side effects

Side effects were evaluated in all patients. Safety measurements included the monitoring of adverse events throughout the study. In addition, vital signs (heart rate, blood pressure), eye examination, urinalysis, and blood chemistry (creatinine, albumin, total bilirubin, alanine transaminase ALT, aspartate transaminase AST) were assessed. Haematology analysis (complete blood count) was also performed. Safety measurements were performed at screening, at baseline and at days 28 and 56 of treatment.

### Other antipsoriasis treatments

None were authorized at the exception of tar- or salicylic acid-based shampoos or of weak corticosteroids for application on facial lesions or genital organs.

A hydrating cream was provided to each participant for the duration of the trial. The participant were allowed to use the cream whenever needed, except during the 24h-period before each visit.

### Medication for treatments of conditions other than psoriasis

All other medication shall remain constant during the trial. If for any reason these treatments should change, the modifications were recorded in the medical dossier of the patients.

### CLINICAL RESULTS

A total of 11 patients, 7 males and 4 females, met the inclusion criteria and were enrolled in the study. All 11 patients completed the initial 56 days study. The age of patients ranged from 27 to 69 years old. The PASI score at baseline ranged from 5.2 to 17.4 and the BSA with psoriasis at baseline ranged from 2 to 20 %. Seven (7) out of the 11 patients were available and agreed to participate to the additional 8 weeks-extension study.

### Changes in psoriasis symptoms

### PASI scores

After one month of treatment, XP-828L improved the score of psoriasis area-and severity index in 6 out of 11 subjects (55 %). At the end of the 56 days study, 7 of the 11 subjects (64 %) had an improvement of their PASI score ranging from 9.5 to 81.3 %. One patient achieved PASI 75 (at least 75 % improvement in PASI relative to baseline) at day 56 of treatment. PASI 75 is the currently recognized benchmark of end points used in psoriasis clinical trials. One patient experienced an increase in its PASI score of 13.6 %. For the other 3 patients, the PASI score remained unchanged. (Table 2).

**Table 1 Summary of the assessments at each patient's visit.**

| | Day of the visit | | | | | | |
|---|---|---|---|---|---|---|---|
| | (screening) -30 | (baseline) 1 | 7 | 28 | 56 | 84 | 112 |
| Written informed consent | x | | | | | | |
| Review of criteria | x | x | x | x | x | x | x |
| Examination of skin | x | | | | | | |
| Blood pressure | x | x | x | x | x | x | x |
| Pregnancy test | x | x | x | x | x | x | x |
| Blood sample (hematology & biochemistry) | x | x | x | x | x | x | x |
| Examen du fond de l'oeil | x | | | | | | x |
| Prosiasis evaluation | | | | | | | |
| %BSA | | x | | x | x | x | x |
| PASI | | x | | x | x | x | x |
| PGA | | x | | x | x | x | x |
| Patient GA | | x | | x | x | x | x |
| Prurit | | x | | x | x | x | x |
| Photographs | | x | | x | x | x | x |
| Report of adverse effects | | | x | x | x | x | x |

**Table 2 PASI scores and changes in PASI score (%) of subjects with psoriasis treated for 56 days (11 patients) or 112 days (7 patients) with 5 g twice daily of XP-828L.**

| | **PASI score** | | | | | **Changes in PASI score (%)** | | |
|---|---|---|---|---|---|---|---|---|
| **Patient** ^{**b**} | **1** | **Day 28** | **Day 56** | **Day 84** | **Day 112** | **Day 56 relative to baseline %** | **Day 84 relative to baseline %** | **Day 112 relative to baseline %** |
| **1** | 9 | 9 | 9 | 8.4 | 8.4 | 0 | -6.7 | -6.7 |
| **2**^{**a**} | 7.2 | 6.4 | 4.8 | - | - | -33.3 | - | - |
| **3** | 14.2 | 14.2 | 14.2 | 14.2 | 14.1 | 0 | 0 | -0.7 |
| **4** | 10.2 | 7.8 | 7.8 | 2.9 | 2.2 | -23.5 | -71.6 | -78.4 |
| **6** | 8.4 | 7.6 | 7.6 | 7.6 | 5.6 | -9.5 | -9.5 | -33.3 |
| **7**^{**c**} | 7.6 | 8.5 | 8.8 | - | - | +15.8 | - | - |
| **8** | 5.2 | 4.9 | 3.7 | 3.9 | 3.7 | -28.8 | -25.0 | -28.8 |
| **9** | 17.4 | 10.3 | 7.4 | 6.6 | 6.6 | -57.5 | -62.1 | -62.1 |
| **10**^{**c**} | 10.8 | 10.4 | 8.9 | - | - | -17.6 | - | - |
| **12**^{**c**} | 5.9 | 4.3 | 1.1 | - | - | -81.3 | - | - |
| **13**^{**d**} | 11.4 | 11.4 | 11.4 | 11.4 | 11.4 | 0 | 0 | 0 |
| **Mean** | | | | | | -21.4±28.3 | -25.0±29.9 | -30±30.8 |
| **n** | | | | | | 11 | 7 | 7 |

| | | | | | | | | |
|---|---|---|---|---|---|---|---|---|
| ^{*a*} Patient 2 was excluded from extension study (at day 72) because of a dental surgery | | | | | | | | |
| ^{*b*} Patients 5 and 11 were excluded at the beginning of the study | | | | | | | | |
| ^{*c*} Patients have refused to participate to the extension study | | | | | | | | |

At day 84 in the extension study, 5 patients out of 7 had an improvement of the PASI score relative to baseline ranging from 6.7 to 71.6 %. 3 patients of 7 had smaller PASI scores at day 84 compared to day 56 of treatment. At the end of the extension study, at 112 days of treatment, PASI score improvement ranged from 6.7 to 78.4 %. Another patient reached PASI 75 after 112 days of treatment. 2 patients out of 7 had a further significant reduction of their PASI score at day 112 compared to day 84 of treatment.

### Body surface area

The body surface area affected by psoriasis didn't change significantly during the trial (Table 3).

**Table 3 Changes in body surface area (BSA, %) during the trial.**

| | **BSA (%)** | | | | |
|---|---|---|---|---|---|
| | **Day 1** | **Day 28** | **Day 56** | **Day 84** | **Day 112** |
| **Patient 1** | 6 | 6 | 6 | 6 | 6 |
| **Patient 2** | 6 | 6 | 6 | 6* | - |
| **Patient 3** | 11.5 | 11.5 | 11.5 | 13.5 | 13.5 |
| **Patient 4** | 8 | 8 | 8 | 5 | 5 |
| **Patient 6** | 6 | 6 | 6 | 6 | 6 |
| **Patient 7** | 6.5 | 7 | 7.5 | - | - |
| **Patient 8** | 2 | 1.75 | 1.75 | 1.75 | 1.75 |
| **Patient 9** | 11.5 | 11.5 | 11.5 | 11.5 | 11.5 |
| **Patient 10** | 6 | 6 | 5.8 | - | - |
| **Patient 12** | 3.75 | 3.75 | 1.5 | - | - |
| **Patient 13** | 20 | 20 | 20 | 20 | 20 |
| **Mean±std** | 7.9±4.9 | 7.95±4.9 | 7.8±5.2 | 8.7±5.9 | 9.1±6.2 |

| | | | | | |
|---|---|---|---|---|---|
| * Patient 2 was excluded from extension study (at day 72) because of a dental surgery | | | | | |

### Psoriasis global assessment

The PGA decreased for 2 patients (patient 4 and patient 9) after one month of treatment, and for 2 patients (patient 8 and patient 12) after 2 months of treatment by one point. Two patients considered that their psoriasis improved significantly with XP-828L, showed by the important decrease of the Patient GA (from 10 to 5 for patient 4 and from 6 to 4 for patient 13 at 28 days of treatment). (Table 4 and Table 5).

In the extension study, the PGA and the Patient GA further decreased for patient 4 at day 112. The patient 4 continued to consider that his psoriasis was very improved (Patient GA from 10 at baseline to 3 at day 112).

**Table 4 Changes in physician's global assessment of psoriasis (PGA) during the trial.**

| | **PGA** | | | | |
|---|---|---|---|---|---|
| | **Day 1** | **Day 28** | **Day 56** | **Day 84** | **Day 112** |
| **Patient 1** | 3 | 3 | 3 | 3 | 3 |
| **Patient 2** | 3 | 3 | 3 | 3 * | - |
| **Patient 3** | 3 | 3 | 3 | 3 | 3 |
| **Patient 4** | 3 | 2 | 2 | 2 | 1 |
| **Patient 6** | 3 | 3 | 3 | 3 | 3 |
| **Patient 7** | 3 | 3 | 3 | - | - |
| **Patient 8** | 3 | 3 | 2 | 3 | 3 |
| **Patient 9** | 4 | 3 | 3 | - | - |
| **Patient 10** | 3 | 3 | 3 | - | - |
| **Patient 12** | 2 | 2 | 1 | - | - |
| **Patient 13** | 3 | 3 | 3 | 3 | 3 |

| | | | | | |
|---|---|---|---|---|---|
| * Patient 2 was excluded from extension study (at day 72) because of a dental surgery | | | | | |

**Table 5 Changes in patient's global assessment of psoriasis (Patient GA) during the trial.**

| | **Patient GA** | | | | |
|---|---|---|---|---|---|
| | **Day 1** | **Day 28** | **Day 56** | **Day 84** | **Day 112** |
| **Patient 1** | 7 | 5 | 6 | 7 | 5 |
| **Patient 2** | 7 | 6 | 7 | 6* | - |
| **Patient 3** | 6 | 6 | 6 | 8 | 8 |
| **Patient 4** | 10 | 5 | 5 | 5 | 3 |
| **Patient 6** | 6 | 6 | 6 | 6 | 6 |
| **Patient 7** | 9 | 10 | 10 | - | - |
| **Patient 8** | 3 | 3 | 3 | 3 | 3 |
| **Patient 9** | 5 | 8 | 5 | 7 | 7 |
| **Patient 10** | 8 | 8 | 10 | - | - |
| **Patient 12** | 2 | 3 | 2 | - | - |
| **Patient 13** | 6 | 4 | 4 | 5 | 5 |

| | | | | | |
|---|---|---|---|---|---|
| * Patient 2 was excluded from extension study (at day 72) because of a dental surgery | | | | | |

### Pruritus

The pruritus scale did not change significantly during the trial. Tree patients experienced a reduction of their pruritus Table 6).

**Table 6 Changes in pruritus reported by the patients during the trial.**

| | **Pruritus** | | | | |
|---|---|---|---|---|---|
| | **Day 1** | **Day 28** | **Day 56** | **Day 84** | **Day 112** |
| **Patient 1** | 1 | 1 | 1 | 1 | 1 |
| **Patient 2** | 1 | 1 | 1 | 1 * | - |
| **Patient 3** | 1 | 1 | 1 | 1 | 1 |
| **Patient 4** | 2 | 2 | 1 | 1 | 1 |
| **Patient 6** | 2 | 1 | 1 | 1 | 1 |
| **Patient 7** | 1 | 2 | 3 | - | - |
| **Patient 8** | 3 | 2 | 3 | 3 | 3 |
| **Patient 9** | 1 | 1 | 0 | 1 | 1 |
| **Patient 10** | 2 | 3 | 2 | - | - |
| **Patient 12** | 1 | 1 | 1 | - | - |
| **Patient 13** | 1 | 1 | 1 | 0 | 0 |

| | | | | | |
|---|---|---|---|---|---|
| * Patient 2 was excluded from extension study (at day 72) because of a dental surgery | | | | | |

### Changes in hematology & biochemical measurements

XP-828L was well tolerated. No clinically significant laboratory abnormalities or pattern of changes in vital signs were observed during the XP-828L treatment.

Table 7 reports the data for creatinine, ALT and AST. It can be noticed that two ALT values for patient 3 were out of range at days 28 (ALT=63) and 56 (ALT=62). These fluctuations were not considered significant since all other values for patient 3 were within ranges.

Also, two patients (patients 12 and 13) experienced not clinically significant temporary fluctuations in creatinine values during the trial. Appendix 3 reports all these fluctuations in creatinine which occurred only once for patient 13 but on three times for patient 12. In each case, a new blood test 2-4 days later showed creatinine values back to normal.

### Adverse events

No clinically significant adverse events were reported during the treatment.

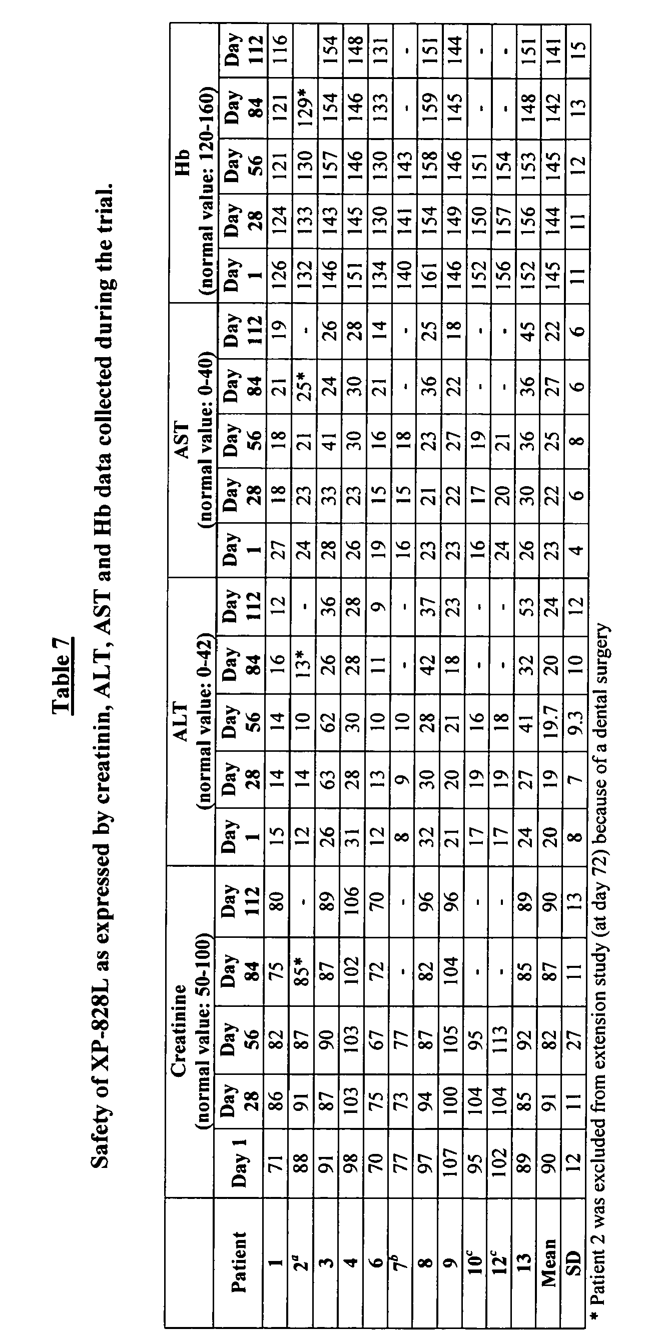

### Efficacy of XP-828L

In this open label study on patients with mild-to-moderate chronic plaque psoriasis, XP-828L treatment for 56 days showed to improve psoriasis. Scores for the psoriasis area-and severity index started to decrease after one month of treatment which can be considered a remarkable achievement for a nutraceutical product. Two patients have reached PASI 75, one after 56 days of treatment and one after 112 days of treatment.

The extent of PASI scores reduction was generally lower during the extension period. As shown in Table 2, the average decrease in PASI scores was 21.5% at day 56 and -25.0 at day 112. The other parameters (PGA, patient's global assessment, pruritus) remained stable during the extension study. This suggests that the efficacy of XP-828L has reached its maximum after day 56. It also shows that the improvements of XP-828L on psoriasis are preserved during the extension period.

No patient experienced a complete elimination of the psoriasis symptoms, suggesting perhaps that a higher dose would lead to more important effects in terms of PASI scores reduction.

### Safety of XP-828L

Since there were no clinical significant side effects or adverse events with the use of XP-828L for 112 days, it is suggested that XP-828L may represent a safe way to treat psoriasis. Only one patient experienced fluctuations of his creatinine values but no fluctuation pattern was found and no other abnormalities were observed.

### The main findings of this open label study are:

XP-828L showed to have the potential of improving psoriasis on patients with mild-to-moderate chronic plaque psoriasis. Scores for the psoriasis area-and severity index already started to decrease after one month of treatment.

No clinically significant adverse events or laboratory abnormalities were observed during the XP-828L treatment. This results suggest that XP-828L is safe.

Overall, the tolerability of XP-828L was good. Patients reported that they liked the product and found its use very convenient.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. A composition for prophylaxis or treatment of psoriasis or related disorders, said composition comprising TGF-β1, TGF-β2, and a total protein concentration completed to at least 15% (w/w) with dairy derived proteins.

2. Use of TGF-β1 and TGF-β2, and dairy derived proteins in the preparation of a composition for preventing or treating psoriasis or related disorders, said dairy derived proteins being at a concentration of at least 15% (w/w).

3. Anyone of claims 1 or 2, wherein said dairy derived proteins are in a concentration of at least 50% (w/w).

4. Anyone of claims 1 or 2, wherein said dairy derived proteins are in a concentration of at least 80% (w/w).

5. Anyone of claims 1 or 2, wherein said TGF-β1 is at concentration between 0.1 to 5 µg/g, and said TGF-β2 at concentration between 5 to 50 µg per gram of composition.

6. Anyone of claims 1 or 2, wherein said TGF-β1 is at concentration between 0.2 to 1.2 µg/g, and said TGF-β2 at concentration between 10 to 18 µg per gram of composition.

7. Anyone of claims 1 or 2, comprising 0.1 to 15% of minerals.

8. Anyone of claims 1 or 2, wherein 70% of said proteins are hydrosoluble.

9. Anyone of claims 1 or 2, wherein said TGF-β1 and said TGF-β2 are dairy derived.

10. Anyone of claims 1 or 2, wherein said psoriasis or related disorders is at least one of nail psoriasis, plaque psoriasis, guttate psoriasis, erythrodermic psoriasis, pustular psoriasis, hyperkeratosis, onycholysis, or psoriatic arthritis.

11. Anyone of claims 1 or 2, comprising IGF-1.

12. Anyone of claims 1 or 2, wherein ratio of TGF-β1/ + TGF-β2/IGF-1 is of between 0.1 to 3.
